(19) Europäisches Patentamt
European Patent Office
Office européen des brevets

(11) Publication number: **0 162 388**
**B1**

(12) **EUROPEAN PATENT SPECIFICATION**

(45) Date of publication of patent specification: **13.09.89**

(21) Application number: **85105788.5**

(22) Date of filing: **10.05.85**

(51) Int. Cl.$^4$: **C 08 F 8/44**, C 08 F 226/06, A 61 K 31/785 // (C08F226/06, 220:20)

(54) **Novel bile sequestrant resin and uses.**

(30) Priority: **11.05.84 US 609269**
**11.05.84 US 609408**

(43) Date of publication of application:
**27.11.85 Bulletin 85/48**

(45) Publication of the grant of the patent:
**13.09.89 Bulletin 89/37**

(84) Designated Contracting States:
**AT BE CH DE FR GB IT LI LU NL SE**

(56) References cited:
**FR-A-2 379 286**
**GB-A-2 011 912**
**GB-A-2 093 041**
**GB-A-2 093 848**

(73) Proprietor: **Bristol-Myers Company**
**345 Park Avenue**
**New York New York 10154 (US)**

(72) Inventor: **Pircio, Anthony W.**
**135 Stanwood Lane**
**Manlius, NY (US)**
Inventor: **Buyniski, Joseph P.**
**302 Rugby Road**
**Syracuse, NY (US)**
Inventor: **Comer, William T.**
**7 Stag Lane**
**Greenwich, CT (US)**
Inventor: **Johnson, Porter C.**
**276 South El Molino Avenue**
**Pasadena, CA (US)**

(74) Representative: **Kinzebach, Werner, Dr.**
**Patentanwälte Reitstötter, Kinzebach und Partner Sternwartstrasse 4 Postfach 86 06 49**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to novel resins useful to sequester non-absorbed bile acids from the intestinal tract to form a complex excreted in the feces with the consequent effect of lowering blood cholesterol level. Also, some of these resins are additionally very useful in inhibiting diarrhea.

It is known to use sequestering agent resins to bind non-absorbed bile acids in the intestinal tract to form complexes which are excreted in the feces whereby bile acids which would otherwise be reabsorbed and returned to the liver are removed from the system. This interrupts the enterohepatic cycle and leads to increased oxidation of cholesterol to bile acids and reduction in plasma cholesterol level. Such sequestering agent resins have been recognized for use for treatment of hypercholesterolemia, especially primary hypercholesterolemia (i.e. elevated low density lipoproteins). Recently, it has been recognized that reducing serum cholesterol has a beneficial effect on protecting against the occurrence of atheroschlerosis including heart disease. Thus, sequestering agent resins for bile acids have recently assumed increased importance.

Cholestyramine is one sequestering agent resin currently being used to lower blood cholesterol levels in humans by binding bile acids in the intestinal tract. Since it is orally administered, the fact that it has an undesirable odor and taste requires use in combination with masking excipients. Moreover, while cholestyramine is quite effective in binding bile acids *in vitro*, binding at between 50 and 80% of its theoretical capacity, *in vivo* much of the bile acid is stripped off in the ileum so that cholestyramine has a low gram potency. This low gram potency in combination with bulk added by the need for masking excipients has resulted in need for a daily intake of 27—54 grams per day which has inhibited widespread use of the drug.

Colestipol is another resin orally administered to sequester bile acids to reduce blood cholesterol level. It has a potency about 50% of cholestyramine.

GB—A—2 093 041 provides a special kind of quaternized vinylimidazole polymers used as a mordant for dyes in photographic colour diffusion transfer processes, containing repeating units of 1-vinylimidazole and/or a 2-alkyl-1-vinylimidazole partially quaternized with 1-chloromethyl-carbonyl-2,3-dihydroxypropane. Contrary to the present invention these polymers are not cross-linked and show solubility in water. Therefore these compounds are not suitable for use as sequestrants according to the present invention.

GB—A—2 093 848 provides a cholesterol reducing polymer composed of monomers of the general formula:

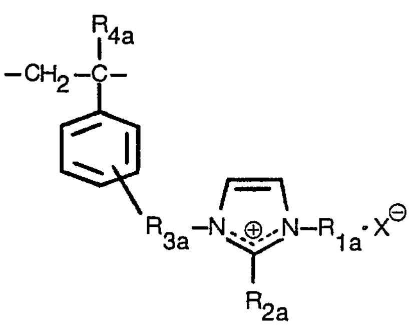

optionally crosslinked with ethylene glycol dimethacrylate. Contrary to the present invention these compounds contain an additional aromatic ring. Furthermore these polymers are not suitable for treating diarrhea.

Recently sequestering agent resins, including cholestyramine and colestipol, have been considered to protect against or treat diarrhea. Numerous clinical reports during the past 10 years have shown cholestyramine to be beneficial in treatment of certain chronic infantile diarrheas. This alternative is significant because infantile diarrhea leads to water and essential salt loss with the consequent risk of dehydration and the normal treatment of hdyration and of essential salts sometimes is ineffective. Oral administration of cholestyramine has also been beneficial in instances where diarrhea is considered to be caused by the cathartic effect of bile acids reaching the colon in increased amounts, such as occurs in ileal resection and by-pass. There is also some indication that cholestyramine may be useful in the treatment of bacterial-induced diarrhea by binding bacterial enterotoxins.

### Summary of the Invention

It has been discovered herein that particular quaternized vinylimidazole-ethylene glycol dimethacrylate copolymers are bile acid sequestering resins which are alternatives for cholestyramine and colestipol.

These copolymers contain from 1% to 25% of ethylene glycol dimethacrylate moieties. The term ethylene glycol dimethacrylate moieties is used herein to mean the portions of the copolymer structure derived from ethylene glycol dimethacrylate. The percentages of ethylene glycol dimethacrylate copolymer moieties herein are weight percentages of the copolymer.

The remainder of these copolymers (referred to hereinafter as copolymer I) consists of polyvinylimidazolium moieties having the structural formula

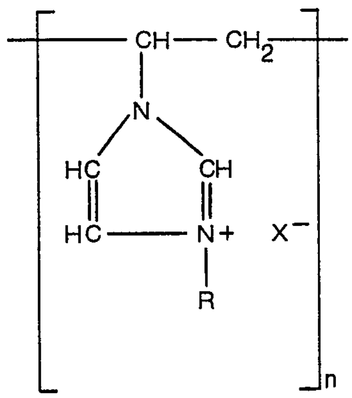

wherein n, on average, ranges from 3 to 40 and wherein R is selected from the group consisting of alkyl and alkenyl groups having from 1 to 18 carbon atoms and $—CH_2R^1$ wherein $R^1$ is selected from the group consisting of phenyl, $—CH_2OH$, $—CH_2NHCOC_6H_5$, and $—CH_2NR^2R^3$ wherein $R^2$ and $R^3$ are each selected from the group consisting of methyl and ethyl and wherein $X^-$ is an anion. The term "polyvinylimidazolium moiety" is used herein to refer to each portion of the copolymer with the structural formula as depicted above, and n is given on the basis of average values because a plurality of such moieties can be found in a copolymer structure. The average value for n is readily obtained by considering the amount of ethylene glycol dimethacrylate in the copolymer and the number of vinyl groups in the copolymer structure.

The present invention also relates to a process for preparing these copolymers. Said process comprises:

(a) reacting 1-vinylimidazole monomer and ethylene glycol dimethacrylate monomer in the presence of a suitable polymerization catalyst under polymerization conditions so as to form a first polymerization product; and then

(b) quaternizing said first polymerization product so as to form said copolymer.

Preferred resins referred to hereinafter as copolymers II, which possess about 130 to 160% of the potency of ground cholestyramine in respect to sequestering bile acids in the intestinal tract of rats to form complexes which are excreted in the feces, are the copolymers I which contain from 15% to 20% of ethylene glycol dimethacrylate moieties and wherein in the above set forth structural formula n averages from 3 to 10 wherein R is alkenyl having from two to four carbon atoms.

As indicated above, the copolymers I and II have utility as sequestrants for bile acids in mammals and are orally administered to said mammals in an amount effective to increase the amount of bile acids in the feces. The copolymers function by binding non-absorbed bile acids in the intestinal tract to form complexes which are excreted in the feces. It is well recognized that removal of bile acids from the intestinal tract in this way lowers blood cholesterol level.

It has been discovered herein that particular copolymers I, referred to hereinafter as copolymers III, are useful to inhibit diarrhea in mammals, i.e. as a prophylactic against the occurrence of diarrhea or as treatment for diarrhea. The copolymers III contain from 1% to 19% of ethylene glycol dimethacrylate and have polyvinylimidazolium moieties with the above set forth structural formula where n averages from 25 to 35 and wherein R is alkyl having from 1 to 3 carbon atoms.

The copolymers III are orally administered in an amount effective to inhibit diarrhea.

Preferred copolymers of the present invention contain from 2% to 5% by weight ethylene glycol dimethacrylate moieties with R being preferably methyl.

Another preferred embodiment provides polyvinylimidazolium moieties with n averaging from 28 to 32 and with $X^-$ being preferably a halide.

Most preferred copolymers contain polyvinylimidazolium moieties with n averaging 30 and with $X^-$ being most preferably chloride.

A further aspect of the present invention provides a pharmaceutical composition which comprises at least one of the copolymers I to III optionally together with pharmaceutical carriers and/or adjuvants.

The copolymers I, II and III herein, as indicated in more detail hereinafter are reaction products wherein one reactant is ethylene glycol dimethacrylate. The ethylene glycol dimethacrylate can be considered a cross-linking agent with potential sites depicted below:

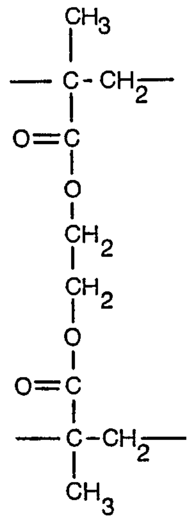

In view of this, the ethylene glycol dimethacrylate (EGD) is sometimes referred to hereinafter as a cross-linking agent.

Turning now to the variables in the resin structure, quaternization is critical because resin which is the same as that of the invention except unquaternized has very low bile acid binding activity. Furthermore, the selection of R in the copolymer structure set forth above is a variable in respect to bile acid binding activity. Furthermore, it has been found herein that selection of particular R and n in the copolymer structure set forth above and particular percentage EGD cross-linker provides a very high order of bile acid binding activity. Moreover, it has also been found that selection of particular R and n and of ethylene glycol dimethacrylate in place of other reactant/constituent and of the percentage thereof determines anti-diarrheal activity.

So far as $X^-$ is concerned in the above structural formula used in conjunction with describing copolymer I, a wide variety of anions are useful herein, a criterion being the pharmaceutically acceptable nature of such. Suitable anions include, for example, halides, acetate, propionate and phosphate. Chloride is preferred.

As indicated above, the copolymers I have bile acid binding activity. The copolymers II and III herein are species of copolymers I. The copolymers II are very superior bile acid binders and even significantly surpass cholestyramine in this regard. The copolymers III, in addition to having bile acid binding capacity, are excellent anti-diarrheal agents.

Examples of copolymers I which are not copolymers II or III are listed below. These have the structure set forth for copolymers I above and variations in R and n in said structure and the percentage ethylene glycol dimethacrylate moieties (% EGD) are set forth below:

| Copolymer | R | n | % EGD |
|---|---|---|---|
| IA | $—CH_3$ | 5 | 17 |
| IB | $—CH_2CH_2CH_3$ | 5 | 17 |
| IC | $—(CH_2)_7CH_3$ | 5 | 17 |
| ID | $—(CH_2)_{15}CH_3$ | 5 | 17 |
| IE | $—CH_2C_6H_5$ | 5 | 17 |
| IF | $—CH_2CH_2OH$ | 5 | 17 |
| IG | $—CH_2CH_2NHCOC_6H_5$ | 4 | 17 |
| IH | $—CH_2CH_2N(C_2H_5)_2$ | 4 | 20 |

Examples of copolymer II are listed below. These have structures as set forth for copolymers II above and the variations in R and n in said structures and the percentage ethylene glycol dimethacrylate moieties (% EGD) are set forth below:

| Copolymer | R | n | % EGD |
|---|---|---|---|
| IIA | $—CH_2CH=CH_2$ | 5 | 17 |
| IIB | $—CH_2CH=CH_2$ | 8 | 16 |
| IIC | $—CH_2CH_2CH=CH_2$ | 3 | 19 |

Copolymer IIA is preferred for bile acid binding/anticholesterol activity.

Examples of copolymers III are listed below. These have structures as set forth for copolymers III above and the variations in R and n in said structures and the percentage ethylene glycol dimethacrylate moieties (% EGD) are set forth below:

| Copolymer | R | n | % EGD |
|---|---|---|---|
| IIIA | $—CH_3$ | 30 | 3 |
| IIIB | $—CH_3$ | 25 | 3 |
| IIIC | $—CH_3$ | 35 | 3 |
| IIID | $—CH_3$ | 28 | 7 |
| IIIE | $—CH_2CH_3$ | 25 | 9 |
| IIIF | $—CH_2CH_2CH_3$ | 33 | 2 |

Copolymer IIIA is preferred for anti-diarrheal activity.

In the above, IA—IH, IIA—IIC and IIA—IIIF, the resins have structure as set forth for copolymer I with $X^-$ as chloride. Useful resins are also provided when the $X^-$ in these exemplary resins is another resin anion as indicated above.

The copolymers I (including species copolymers II and III) are readily prepared, for example, by reacting 1-vinylimidazole and ethylene glycol dimethacrylate and then quaternizing. The reaction of 1-vinylimidazole and ethylene glycol dimethacrylate is readily carried out at, for example, 55°C—80°C, in the presence of benzoyl peroxide catalyst. For copolymers where the ethylene glycol dimethacrylate moiety content is 10% or more a mixture of aqueous sodium sulfate and ethyl acetate is suitable as a reaction medium. Where such content is less than 10%, benzene is suitable as a reaction medium. Quaternization is carried out by refluxing, for example, in ethanol with quaternizing agent. Chloride formation is advantageously carried out by quaternizing with iodide and then exchanging utilizing silver chloride.

For bile acid binding capacity a dosage of 2 to 5 grams three or four times per day is preferred and with the preferred copolymer for this, namely IIA, 2—3 grams three or four times a day.

For anti-diarrheal activity, a dosage of copolymer III of 1—2 grams twice a day for adults and 0.5—1 grams twice a day for pediatric diarrhea is preferred.

The following examples are illustrative of the discoveries herein.

## Example I

Preparation of Copolymer IIA

A freshly prepared solution of 1-vinylimidazole (400 ml, 4.44 moles), ethylene glycol dimethacrylate (40 ml, 0.21 moles), and benzoyl peroxide (38.0 g, 0.16 moles) in ethyl acetate (400 ml) was added to an aqueous solution of sodium sulfate (2.0 l, saturated at 60°C) kept at 60°C. The mixture was stirred well for 16 hours under nitrogen. The resulting beadlets were collected on a filter and washed four times with water. This material was dried two days in a vacuum oven at 80°C to give 142.4 g consisting essentially of vinylimidazole-ethylene glycol dimethacrylate copolymer containing 17% ethylene glycol dimethacrylate moiety and having structural formula set forth above for copolymer I with n equal to 5 except that the resin was unquaternized. 50 g of the resulting product and a solution of allyl chloride (100 ml) in ethanol (400 ml) were combined and refluxed with stirring for 4 days. The mixture was cooled, collected by filtration on crepe paper, and washed thrice with ethanol. The resulting product was dried 18 hours at 60°C in a vacuum oven to 62.30 grams of light brown solid consisting essentially of copolymer IIA as defined hereinbefore.

Example II

Preparation of Copolymer IIIA

Benzene (500 ml) was heated to reflux under nitrogen in a flask. Benzoyl peroxide (27.2 g) was dissolved in another 500 ml of benzene and to this was added 300 ml of 1-vinyl-imidazole and ethylene glycol dimethacrylate (3 ml) to form a solution. This solution was added to the refluxing benzene and washed in with 200 more ml of benzene. The resulting mix was stirred under nitrogen with refluxing for 5 hours. After slight cooling and filtering, the resulting mix was dried under vacuum for 16 hours at 60°C and then dried further under vacuum at 60°C to yield 147.8 g of a viscous liquid including vinylimidazole-ethylene glycol dimethacrylate containing 3% ethylene glycol dimethacrylate moiety and having structure formula as set forth above for copolymer I with an equal to 30 except that the resin was unquaternized. This liquid was admixed with ethanol (1 liter) and iodomethane (90 ml, 1.44 moles), and stirring at room temperature was carried out for 16 hours. The resulting mix was filtered and washed three times with absolute ethanol to provide a light tan powder with a skin on top. The skin was removed and the powder was dried under vacuum at 60°C for 23 hours to yield 127.1 g of light brown powder. To 123.4 g of this was added 40 ml of iodomethane and 500 ml of absolute ethanol, and this mix was refluxed for 19 hours, cooled, filtered, washed three times with absolute ethanol and dried at 60°C under vacuum for 44 hours to yield 137.2 g of poly-3-methyl-1-vinylimidazolium iodide resin. Chloride was then exchanged with iodide as follows: Silver nitrate (144.2 g) was dissolved in water. Sodium chloride (50.8 g) was dissolved in water. The solutions were combined. The resulting silver chloride precipitate was filtered and washed. The washed silver chloride precipitate was combined with the 137.2 g of the resin and 1 l of water. The mixture was stirred well and refluxed for 18 hours. The reaction mixture was centrifuged and the solid was washed with water and centrifuged again. The combined supernatants were concentrated under vacuum at approroximately 90°C. Then ethanol was added and concentration under vacuum carried out. This was repeated twice more. Then acetone was added and solid was scraped off the sides of the flask, filtered, dried 17 hours at 72°C under vacuum and sieved through a #9 screen (aperture of sieve: 1.981 mm) to yield 96.1 g of copolymer IIIA.

Example III

Testing for Bile Acid Binding Activity

Compositions tested herein were copolymers IA, IB, IC, ID IE, IF, IG, IH, IIA and IIIA as described hereinbefore as well as copolymer IVA (the unquaternized precursor of copolymer IA).

The compositions were tested *in vitro* as well as *in vivo*.

The *in vitro* testing consisted of incubating 20 mg amounts of dry resin in 5 ml of 0.15 M NaCl containing 100 µmoles of sodium cholate for 16 hours of continuous shaking at 37°C. After centrifugation, unbound bile salt remaining in the supernatant was measured by the hydroxy-steroid dehydrogenase assay. Results are reported in terms of percentage of the activity of cholestryramine beadlets.

The *in vivo* testing was carried out in rats as follows: Male weanling rats (Wistar strain) were individually housed and fed a basal diet (20.0% casein, 62.55% Amidex®, 10.0% corn oil, 4% non-nutritive fiber, 2.45% mineral mixture and 1.0% vitamin mixture) for a 10 day period. On the basis of body weight and the 10-day weight gain, the animals were selected into groups of 10 animals each and fed the basal diet plus the test resins at 0.25 to 1.0% by weight of the diet for a 14-day test period. During the last four days of the test period feces were collected, freeze-dried, ground and analyzed for bile acids by the hydroxysteroid dehydrogenase enzyme method. Results are reported as a percentage of the activity of cholestyramine fed in the diet at the same percentage value as the test resin.

Test Results for Example III

| Copolymer | In Vitro | In Vivo |
|---|---|---|
| IA | 93 | 113[2], 126[1] |
| IB | 97 | 92[1] |
| IC | 94 | 91[1], 78[2], 88[3] |
| ID | 75 | 61[1], 49[2] |
| IE | 102 | 113[4], 137[5] |
| IF | 107 | 103[5] |
| IG | 82 | 78[1], 80[2] |
| IH | 139 | 79[1], 76[2] |
| IIA (Lot 1) | 119, 111 | 110[4], 135[5] |
| IIA (Lot 2) | 117 | 98[1], 116[2], 121[3], 131[4], 143[4], 161 |
| IIIA | 101 | 52[1], 51[2] |
| IVA (reference-unquaternized) | 3 | 19[1] |

[1]: Compared to cholestyramine beadlets at 1% of diet.
[2]: Compared to cholestyramine beadlets at 0.5% of diet.
[3]: Compared to cholestyramine beadlets at 0.25% of diet.
[4]: Compared to ground cholestyramine at 1% of diet.
[5]: Compared to ground cholestyramine at 0.5% of diet.

As indicated above, the copolymers herein contain at least about 50% of the activity of cholestyramine and preferred copolymer herein (IIA) is from 98% to 161% as effective as cholestyramine. Note also the data for reference resin IVA which indicates unquaternized copolymer has very low activity.

Example IV

Testing for Anti-Diarrhea Activity

Compositions tested herein were the same as tested in Example III.

The compositions were tested *in vivo* in rats as follows: Male Sprague-Dawley rats (150—290 g) were dosed orally with test material 1 hour before receiving a standard dose of castor oil orally (3 ml/rat). The rats were individually caged and exmained for the presence of diarrhea at hourly intervals. Control animals had a 100% incidence of diarrhea 1 hour after castor oil administration.

With a dosage of 100 mg of resin per kilogram of body weight, test results were as follows: Copolymers IA, IB, ID, IE, IF, IH, IIA and IVA did not protect against diarrhea in any rats as of 1 hour after castor oil challenge. Copolymers IC and IG protected 17% of rats against diarrhea as of 1 hour after castor oil challenge but failed to protect any rats two hours after castor oil challenge. On the other hand, copolymer IIIA protected 100% of rats against diarrhea 1 hour after castor oil challenge and 17% of rats even two hours after castor oil challenge.

Copolymer IIIA was tested against cholestyramine and colestipol with results as set forth in the following table:

TABLE — Example IV

| Test Compound | No. of Rats | Dose mg/kg (p.o.) | Percentage of Rats Protected Against Diarrhea | | | | | |
|---|---|---|---|---|---|---|---|---|
| | | | 1 | 2 | 3 | 4 | 5 | 6 Hrs |
| Copolymer IIIA | 6 | 512 | 100 | 100 | 100 | 100 | 100 | 100 |
| | 12 | 256 | 100 | 100 | 100 | 100 | 58 | 17 |
| | 12 | 128 | 100 | 58 | 50 | 33 | 0 | |
| | 12 | 64 | 67 | 25 | 8 | 8 | 0 | |
| | 12 | 32 | 17 | 8 | 0 | | | |
| | 6 | 16 | 0 | | | | | |
| Cholestyramine | 6 | 512 | 100 | 17 | 0 | | | |
| | 12 | 256 | 67 | 8 | 0 | | | |
| | 12 | 128 | 50 | 0 | | | | |
| | 18 | 64 | 33 | 0 | | | | |
| | 18 | 32 | 28 | 8 | | | | |
| | 12 | 16 | 0 | | | | | |
| Colestipol | 6 | 512 | 83 | 0 | | | | |
| | 6 | 256 | 50 | 0 | | | | |
| | 6 | 128 | 17 | 17 | 17 | 17 | 17 | 17 |
| | 6 | 64 | 33 | 0 | | | | |
| | 6 | 32 | 0 | | | | | |
| | 6 | 16 | 0 | | | | | |

The above results indicate that copolymer IIIA at higher dosages protects up to at least 6 hours, and that it is significantly more potent than either cholestyramine or colestipol.

The data indicates an ED50 for copolymer IIIA of 50.8 (38.5—69.3) compared to ED50's for cholestyramine of 107 (68—194) and for colestipol of 213 (118—530).

While the foregoing describes preferred embodiments, modifications within the scope of the invention will be readily evident to those skilled in the art. For example, the resins herein are readily combined with a pharmaceutically acceptable carrier or excipient. Thus, the scope of the invention is intended to be defined by the claims.

**Claims for the Contracting States: BE CH DE FR GB IT LI LU NL SE**

1. Quaternized vinylimidazole-ethylene glycol dimethacrylate copolymers comprising 1% to 25% by weight of ethylene glycol dimethacrylate moieties, the remainder being polyvinylimidazolium moieties having the structural formula

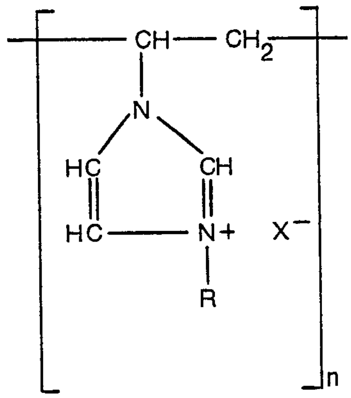

wherein n, on average, ranges from 3 to 40 and wherein R is selected from the group consisting of alkyl and alkenyl groups having from 1 to 18 carbon atoms and $—CH_2R^1$ wherein $R^1$ is selected from the group consisting of phenyl, $—CH_2OH$, $—CH_2NHCOC_6H_5$, and $—CH_2NR^2R^3$ wherein $R^2$ and $R^3$ are each selected from the group consisting of methyl and ethyl and wherein $X^-$ is an anion.

2. Quaternized vinylimidazole-ethylene glycol dimethacrylate copolymer as recited in Claim 1, said copolymer containing from 15% to 20% by weight of ethylene glycol dimethacrylate moieties and wherein the polyvinylimidazolium moieties have n averageing from 3 to 10 and wherein R is alkenyl having from two to four carbon atoms.

3. Quaternized vinylimidazole-ethylene glycol dimethacrylate copolymer as recited in Claim 2, said copolymer containing 17% by weight of ethylene glycol dimethacrylate moieties and wherein the polyvinylimidazolium moieties have n averaging 5 and wherein R is prop-2-en-1-yl, and $X^-$ is chloride.

4. Quaternized vinylimidazole-ethylene glycol dimethacrylate copolymer as recited in Claim 1, said copolymer containing from 1% to 19% by weight of ethylene glycol dimethacrylate moieties and wherein the polyvinylimidazolium moieties have n averaging from 25 to 35 and wherein R is alskyl having from 1 to 3 carbon atoms.

5. Quaternized vinylimidazole-ethylene glycol dimethacrylate copolymer as recited in Claim 1, said copolymer containing from 2% to 5% by weight of ethylene glycol dimethacrylate moieties.

6. Quaternized vinylimidazole-ethylene glycol dimethacrylate copolymer as recited in Claim 5 wherein R is methyl.

7. Quaternized vinylimidazole-ethylene glycol dimethacrylate copolymer as recited in Claim 6 wherein the polyvinylimidazolium moieties have n averaging from 28 to 32.

8. Quaternized vinylimidazole-ethylene glycol dimethacrylate copolymer as recited in Claim 7 wherein $X^-$ is halide.

9. Quaternized vinylimidazole-ethylene glycol dimethacrylate copolymer as recited in Claim 8 wherein the polyvinylimidazolium moieties have n averaging 30.

10. Quaternized vinylimidazole-ethylene glycol dimethacrylate copolymer as recited in Claim 9 wherein $X^-$ is chloride.

11. A process for preparing the copolymer of Claims 1—10, characterized in that

(a) 1-vinylimidazole monomer and ethylene glycol dimethacrylate monomer are reacted in the presence of a suitable polymerization catalyst under polymerization conditions so as to form a first polymerization product; and then

(b) said first polymerization product is quaternized so as to form said copolymer.

12. A process according to Claim 11, wherein said polymerization catalyst is benzoyl peroxide.

13. A compound according to any one of Claims 1—10 for use as a drug for sequestering bile acids in a mammal, for lowering blood cholesterol in a mammal or for treating diarrhea in a mammal.

14. A pharmaceutical compound according to Claim 13 for use as a drug for sequestering bile acids or for lowering blood cholesterol, wherein said copolymer contains from 15% to 20% by weight of ethylene glycol dimethacrylate moieties.

15. A compound according to Claim 14, wherein said copolymer contains 17% by weight of ethylene glycol dimethacrylate moieties.

16. A pharmaceutical composition comprising at least one of the compounds of Claims 1—10, optionally together with pharmaceutical carriers and/or adjuvants.

17. The pharmaceutical composition of Claim 16 in unit dosage form for sequestering bile acids in a mammal, said composition comprising a dosage of 2 to 5 grams of a copolymer according to Claim 2 or Claim 3.

18. The pharmaceutical composition of Claim 16 in unit dosage form for lowering the blood cholesterol level of a mammal, said composition comprising a dosage of 2 to 5 grams of a copolymer according to Claim 2 or Claim 3.

19. The pharmaceutical composition of Claim 16 for inhibiting diarrhea in a mammal, said composition comprising a copolymer according to Claim 4 or Claim 5 or Claim 10.

**Claims for the Contracting State: AT**

1. A process for preparing quaternized vinylimidazole-ethylene glycol dimethacrylate copolymers comprising 1% to 25% by weight of ethylene glycol dimethacrylate moieties, the remainder being polyvinylimidazolium moieties having the structural formula

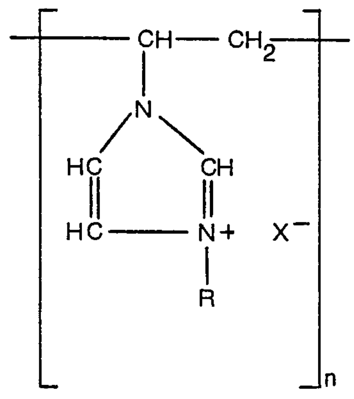

wherein n, on average, ranges from 3 to 40 and wherein R is selected from the group consisting of alkyl and alkenyl groups having from 1 to 18 carbon atoms and $—CH_2R^1$ wherein $R^1$ is selected from the group consisting of phenyl, $—CH_2OH$, $—CH_2NHCOC_6H_5$, and $—CH_2NR^2R^3$ wherein $R^2$ and $R^3$ are each selected from the group consisting of methyl and ethyl and wherein $X^-$ is an anion, characterized in that

(a) 1-vinylimidazole monomer and ethylene glycol dimethacrylate monomer are reacted in the presence of a suitable polymerization catalyst under polymerization conditions so as to form a first polymerization product; and then

(b) said first polymerization product is quaternized so as to form said copolymer.

2. The process of Claim 1 for preparing a quaternized vinylimidazole-ethylene glycol dimethacrylate copolymer, characterized in that said copolymer contains from 15% to 20% by weight of ethylene glycol dimethacrylate moieties and wherein the polyvinylimidazolium moieties have n averaging from 3 to 10 and wherein R is alkenyl having from two to four carbon atoms.

3. The process of Claim 2 for preparing a quaternized vinylimidazole-ethylene glycol dimethacrylate copolymer characterized in that said copolymer contains 17% by weight of ethylene glycol dimethacrylate moieties and wherein the polyvinylimidazolium moieties have n averaging 5 and wherein R is prop-2-en-1-yl, and $X^-$ is chloride.

4. The process of Claim 1 for preparing a quaternized vinylimidazole-ethylene glycol dimethacrylate copolymer, characterized in that said copolymer contains from 1% to 19% by weight of ethylene glycol dimethacrylate moieties and wherein the polyvinylimidazolium moieties have n averageing from 25 to 35 and wherein R is alkyl having from 1 to 3 carbon atoms.

5. The process of Claim 1 for preparing a quaternized vinylimidazole-ethylene glycol dimethacrylate copolymer characterized in that said copolymer contains from 2% to 5% by weight of ethylene glycol dimethacrylate moieties.

6. The process of Claim 5 for preparing a quaternized vinylimidazole-ethylene glycol dimethacrylate, characterized in that R is methyl.

7. The process of Claim 6 for preparing a quaternized vinylimidazole-ethylene glycol dimethacrylate copolymer, characterized in that the polyvinylimidazolium moieties have n averaging from 28 to 32.

8. The process of Claim 7 for preparing a quaternized vinylimidazole-ethylene glycol dimethacrylate copolymer, characterized in that $X^-$ is halide.

9. The process of Claim 8 for preparing a quaternized vinylimidazole-ethylene glycol dimethacrylate copolymer, characterized in that the polyvinylimidazolium moieties have n averaging 30.

10. The process of Claim 9 for preparing a quaternized vinylimidazole-ethylene glycol dimethacrylate copolymer, characterized in that $X^-$ is chloride.

11. A process according to any one of Claims 1—10, characterized in that said polymerization catalyst is benzoyl peroxide.

12. A process according to any one of Claims 1—11 for preparing a pharmaceutical compound for use as a drug for sequestering bile acids or for lowering blood cholesterol, characterized in that said copolymer contains from 15% to 20% by weight of ethylene glycol dimethacrylate moieties.

13. The process of Claim 12 for preparing a pharmaceutical compound, characterized in that said copolymer contains 17% by weight of ethylene glycol dimethacrylate moieties.

14. A process for preparing a pharmaceutical composition, characterized in that at least one of the compounds as defined by Claims 1 to 10 are provided optionally together with pharmaceutical carriers and/or adjuvants.

15. The process of Claim 14 for preparing a pharmaceutical composition in unit dosage form for sequestering bile acids in a mammal, characterized in that the composition comprises a dosage of 2 to 5 grams of a copolymer prepared as defined in Claim 2 or Claim 3.

16. The process of Claim 14 for preparing a pharmaceutical composition in unit dosage form for lowering the blood cholesterol level of a mammal, characterized in that the composition comprises a dosage of 2 to 5 grams of a copolymer prepared as defined in Claim 2 or Claim 3.

17. The process of Claim 14 for preparing a pharmaceutical composition for inhibiting diarrhea in a mammal, characterized in that the composition comprises a copolymer prepared as defined in Claim 4 or Claim 5 or Claim 10.

**Patentansprüche für die Vertragsstaaten: BE CH DE FR GB IT LU LI NL SE**

1. Quaternisierte Vinylimidazol-Ethylenglycoldimethacrylat-Copolymere mit 1 bis 25 Gew.-% Ethylenglycoldimethacrylat-Einheiten, wobei der restliche Anteil aus Polyvinylimidazolium-Einheiten besteht mit folgender Strukturformel:

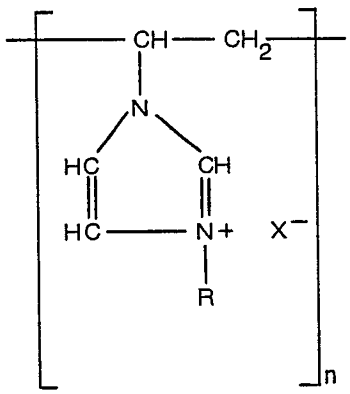

worin

n für einen durchschnittlichen Wert von 3 bis 40 steht und

R ausgewählt ist unter Alkyl- und Alkenylgruppen mit 1 bis 18 Kohlenstoffatomen und dem Rest $CH_2R^1$, worin $R^1$ ausgewählt ist unter Phenyl, $—CH_2OH$, $—CH_2NHCOC_6H_5$ und $—CH_2NH^2R^3$, worin $R^2$ und $R^3$ ausgewählt sind unter einem Methyl- und Ethylrest und worin $X^-$ ein Anion bedeutet.

2. Quaternisiertes Vinylimidazol-Ethylenglycoldimethacrylat-Copolymer nach Anspruch 1, wobei das Copolymer 15 bis 20 Gew.-% Ethylenglycoldimethacrylat-Einheiten enthält und worin die Polyvinylimidazolium-Einheiten einen durchschnittlichen Wert n von 3 bis 10 besitzen und worin R eine Alkenylgruppe mit 2 bis 4 Kohlenstoffatomen ist.

3. Quaternisiertes Vinylimidazol-Ethylenglycoldimethacrylat-Copolymer nach Anspruch 2, wobei das Copolymer 17 Gew.-% Ethylenglycoldimethacrylat-Einheiten enthält und worin die Polyvinylimidazolium-Einheiten einen durchschnittlichen Wert n von 5 besitzen und worin R eine Prop-2-en-1-yl-Gruppe bedeutet und $X^-$ für ein Chloridion steht.

4. Quaternisiertes Vinylimidazol-Ethylenglycoldimethacrylat-Copolymer nach Anspruch 1, wobei das Copolymer 1 bis 19 Gew.-% Ethylenglycoldimethacrylat-Einheiten besitzt und worin die Polyvinylimidazolium-Einheiten einen durchschnittlichen Wert n von 25 bis 35 besitzen und worin R eine Alkylgruppe mit 1 bis 3 Kohlenstoffatomen ist.

5. Quaternisiertes Vinylimidazol-Ethylenglycoldimethacrylat-Copolymer nach Anspruch 1, wobei das Copolymer 2 bis 5 Gew.-% Ethylenglycoldimethacrylat-Einheiten besitzt.

6. Quaternisiertes Vinylimidazol-Ethylenglycoldimethacrylat-Copolymer nach Anspruch 5, worin R für eine Methylgruppe steht.

7. Quaternisiertes Vinylimidazol-Ethylenglycoldimethacrylat-Copolymer nach Anspruch 6, worin die Polyvinylimidazolium-Einheiten einen durchschnittlichen Wert n von 28 bis 32 besitzen.

8. Quaternisiertes Vinylimidazol-Ethylenglycoldimethacrylat-Copolymer nach Anspruch 7, worin $X^-$ ein Halogenidion ist.

9. Quaternisiertes Vinylimidazol-Ethylenglycoldimethacrylat-Copolymer nach Anspruch 8, worin die Polyvinylimidazolium-Einheiten einen durchschnittlichen Wert n von 30 besitzen.

10. Quaternisiertes Vinylimidazol-Ethylenglycoldimethacrylat-Copolymer nach Anspruch 9, worin $X^-$ für ein Chloridion steht.

11. Verfahren zur Herstellung von Copolymeren gemäß den Ansprüchen 1 bis 10, dadurch gekennzeichnet, daß

(a) das 1-Vinylimidazol-Monomer und das Ethylenglycol-dimethacrylat-Monomer in Gegenwart eines geeigneten Polymerisationskatalysators unter Polymerisationsbedingungen umgesetzt werden, so daß ein erstes Polymerisationsprodukt gebildet wird; und anschließend

(b) das erste Polymerisationsprodukt unter Bildung des Copolymers quaternisiert wird.

12. Verfahren nach Anspruch 11, wobei der Polymerisationskatalysator Benzoylperoxid ist.

13. Verbindung nach einem der Ansprüche 1 bis 10 zur Verwendung als Wirkstoff zur Sequestrierung von Gallensäuren in Säugetieren, zur Verminderung des Blutcholesterols in Säugetieren oder zur Behandlung von Diarrhoe in Säugetieren.

14. Pharmazeutische Verbindung nach Anspruch 13 zur Verwendung als Wirkstoff zur Sequestrierung von Gallensäuren oder zur Verminderung des Blutcholesterols, wobei das Copolymer 15 bis 20 Gew.-% Ethylenglycoldimethacrylat-Einheiten aufweist.

15. Verbindung nach Anspruch 14, worin das Copolymer 17 Gew.-% Ethylenglycoldimethacrylat-Einheiten aufweist.

16. Pharmazeutisches Mittel, umfassend mindestens eine der Verbindungen gemäß den Ansprüchen 1 bis 10, gegebenenfalls zusammen mit pharmazeutischen Trägern und/oder Adjuvanzien.

17. Pharmazeutisches Mittel nach Anspruch 16 in einer Dosiseinheit zur Sequestrierung von Gallensäuren in einem Säugetier, wobei das Mittel eine Dosis von 2 bis 5 g eines Copolymers gemäß Anspruch 2 oder Anspruch 3 umfaßt.

18. Pharmazeutisches Mittel nach Anspruch 16 in einer Dosiseinheit zur Verringerung des Blutcholesterolspiegels eines Säugetiers, wobei das Mittel eine Dosis von 2 bis 5 g eines Copolymers gemäß Anspruch 2 oder Anspruch 3 umfaßt.

19. Pharmazeutisches Mittel nach Anspruch 16 zur Inhibierung von Diarrhoe in einem Säugetier, wobei das Mittel ein Copolymer gemäß Anspruch 4 oder Anspruch 5 oder Anspruch 10 umfaßt.

**Patentansprüche für die Vertragsstaat: AT**

1. Verfahren zur Herstellung von quaternisierten Vinylimidazol-Ethylenglycoldimethacrylat-Copolymeren mit 1 bis 25 Gew.-% Ethylenglycoldimethacrylat-Einheiten, wobei der restliche Anteil Polyvinylimidazolium-Einheiten darstellt, die folgende Strukturformel besitzen:

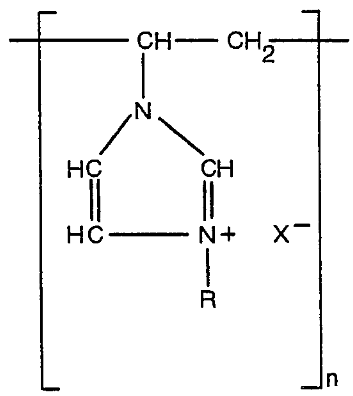

worin

n für einen durchschnittlichen Wert von 3 bis 40 steht und

R ausgewählt ist unter Alkyl- und Alkenylgruppen mit 1 bis 18 Kohlenstoffatomen und dem Rest $CH_2R^1$, worin $R^1$ ausgewählt ist unter Phenyl, $—CH_2OH$, $—CH_2NHCOC_6H_5$ und $—CH_2NH^2R^3$, worin $R^2$ und $R^3$ ausgewählt sind unter einem Methyl- und Ethylrest und worin $X^-$ ein Anion bedeutet, dadurch gekennzeichnet, daß

(a) das 1-Vinylimidazol-Monomer und das Ethylenglycoldimethacrylat-Monomer in Gegenwart eines geeigneten Polymerisationskatalysators unter Polymerisationsbedingungen umgesetzt werden, so daß ein erstes Polymerisationsprodukt gebildet wird; und anschließend

(b) das erste Polymerisationsprodukt unter Bildung des Copolymers quaternisiert wird.

2. Verfahren nach Anspruch 1 zur Herstellung eines quaternisierten Vinylimidazol-Ethylenglycoldimethacrylat-Copolymers, dadurch gekennzeichnet, daß das Copolymer 15 bis 20 Gew.-% Ethylenglycoldimethacrylat-Einheiten enthält und worin die Polyvinylimidazolium-Einheiten enthält und worin die Polyvinylimidazolium-Einheiten einen durchschnittlichen Wert n von 3 bis 10 besitzen und worin R eine Alkylengruppe mit 2 bis 4 Kohlenstoffatomen ist.

3. Verfahren nach Anspruch 2 zur Herstellung eines quaternisierten Vinylimidazol-Ethylenglycoldimethacrylat-Copolymers, dadurch gekennzeichnet, daß das Copolymer 17 Gew.-% Ethylenglycoldimethacrylat-Einheiten enthält und worin die Polyvinylimidazolium-Einheiten einen durchschnittlichen Wert n von 5 besitzen und worin R eine Prop-2-en-1-yl-Gruppe bedeutet und $X^-$ für ein Chloridion steht.

4. Verfahren nach Anspruch 1 zur Herstellung eines quaternisierten Vinylimidazol-Ethylenglycoldimethacrylat-Copolymers, dadurch gekennzeichnet, daß das Copolymer 1 bis 19 Gew.-% Ethylenglycoldimethacrylat-Einheiten besitzt und worin die Polyvinylimidazolium-Einheiten einen durchschnittlichen Wert n von 25 bis 35 besitzen und worin R eine Alkylgruppe mit 1 bis 3 Kohlenstoffatomen ist.

5. Verfahren nach Anspruch 1 zur Herstellung eines quaternisierten Vinylamidazol-Ethylenglycol-

dimethacrylat-Copolymers, dadurch gekennzeichnet, daß das Copolymer 2 bis 5 Gew.-% Ethylenglycoldimethacrylat-Einheiten besitzt.

6. Verfahren nach Anspruch 5 zur Herstellung eines quaternisierten Vinylimidazol-Ethylenglycoldimethacrylat-Copolymers, dadurch gekennzeichnet, daß R für eine Methylgruppe steht.

7. Verfahren nach Anspruch 6 zur Herstellung eines quaternisierten Vinylimidazol-Ethylenglycoldimethacrylat-Copolymers, dadurch gekennzeichnet, daß die Polyvinylimidazolium-Einheiten einen durchschnittlichen Wert n von 28 bis 32 besitzen.

8. Verfahren nach Anspruch 7 zur Herstellung eines quaternisierten Vinylimidazol-Ethylenglycoldimethacrylat-Copolymers, dadurch gekennzeichnet, das $X^-$ für ein Halogenidion steht.

9. Verfahren nach Anspruch 8 zur Herstellung eines quaternisierten Vinylimidazol-Ethylenglycoldimethacrylat-Copolymers, dadurch gekennzeichnet, daß die Polyvinylimidazolium-Einheiten einen durchschnittlichen Wert n von 30 besitzen.

10. Verfahren nach Anspruch 9 zur Herstellung eines quaternisierten Vinylimidazol-Ethylenglycoldimethacrylat-Copolymers, dadurch gekennzeichnet, daß $X^-$ für ein Chloridion steht.

11. Verfahren nach einem der Ansprüche 1 bis 10, dadurch gekennzeichnet, daß der Polymerisationskatalysator Benzoylperoxid ist.

12. Verfahren nach einem der Ansprüche 1 bis 11 zur Herstellung einer pharmazeutischen Verbindung zur Verwendung als Wirkstoff für die Sequestrierung von Gallansäuren oder zur Verminderung des Blutcholesterols, dadurch gekennzeichnet, daß das Copolymer 15 bis 20 Gew.-% Ethylenglycoldimethacrylat-Einheiten besitzt.

13. Verfahren nach Anspruch 12 zur Herstellung einer pharmazeutischen Verbindung, dadurch gekennzeichnet, daß das Copolymer 17 Gew.-% Ethylenglycoldimethacrylat-Einheiten besitzt.

14. Verfahren zur Herstellung eines pharmazeutischen Mittels, dadurch gekennzeichnet, daß mindestens eine der Verbindungen gemäß den Ansprüchen 1 bis 10 gegebenenfalls zusammen mit pharmazeutischen Trägern und/oder Adjuvanzien bereitgestellt wird.

15. Verfahren nach Anspruch 14 zur Herstellung eines pharmazeutischen Mittels in einer Dosiseinheit zur Sequestrierung von Gallensäuren in einem Säugetier, dadurch gekennzeichnet, daß das Mittel eine Dosis von 2 bis 5 g eines Copolymers umfaßt, das gemäß Anspruch 2 oder Anspruch 3 hergestellt wurde.

16. Verfahren nach Anspruch 14 zur Herstellung eines pharmazeutischen Mittels in einer Dosiseinheit zur Verminderung des Blutcholseterolspiegels in einem Säugetier, dadurch gekennzeichnet, daß das Mittel eine Dosis von 2 bis 5 g eines Copolymers umfaßt, das gemäß Anspruch 2 oder Anspruch 3 hergestellt wurde.

17. Verfahren nach Anspruch 14 zur Herstellung eines pharmazeutischen Mittels zur Inhibierung von Diarrhoe in einem Säugetier, dadurch gekennzeichnet, daß das Mittel ein Copolymer umfaßt, welches gemäß Anspruch 4 oder Anspruch 5 oder Anspruch 10 hergestellt wurde.

**Revendications pour les Etats contractants: BE CH DE FR GB IT LI LU NL SE**

1. Copolymères de vinylimidazole quarternisé-diméthacylate d'éthylène glycol comprenant de 1 pour cent à 25 pour cent en poids de motifs diméthacrylate d'éthylène glycol, le reste étant constitué par des motifs polyvinylimidazolium de formule structurelle

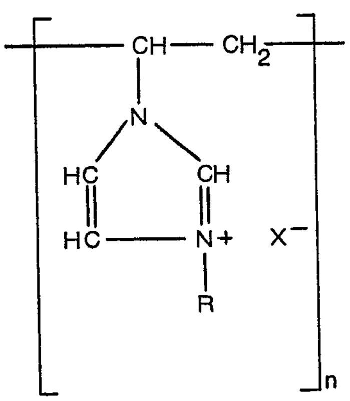

dans laquelle:

n, en moyenne, est de l'ordre de 3 à 40 et

R est choisi dans le groupe formé par les radicaux alkyle et alkényle possédant de 1 à 18 atomes de carbone et $—CH_2R^1$ dans laquelle $R^1$ est choisie dans le groupe formé par les radicaux phényle $—CH_2OH$, $—CH_2NHCOC_6H_5$, et $—CH_2NR^2R^3$ dans lesquels $R^2$ et $R^3$ sont chacun choisis dans le groupe formé par les radicaux méthyle et éthyle et dans lesquels $X^-$ est un anion.

2. Copolymère de vinylimidazole quaternisé-diméthacrylate d'éthylène glycol selon la revendication 1, ledit copolymère contenant de 15 à 20% en poids de motifs diméthacylate d'éthylène glycol et dans lequel

les motifs polyvinylimidazolium présentent une valeur moyenne de n de 3 à 10 et dans lequel R est un radical alkényle ayant de 2 à 4 atomes de carbone.

3. Copolymère de vinylimidazole quaternisé-diméthacrylate d'éthylène glycol selon la revendication 2, ledit copolymère contenant 17% en poids de motifs diméthacrylate d'éthylène glycol et dans lequel les motifs polyvinylimidazolium présentent une valeur moyenne de n de 5 et dans lequel R est un radical propyl-2-ène-1-yle, et $X^-$ est l'ion chlorure.

4. Copolymère de vinylimidazole quarternisé-diméthacrylate d'éthylène glycol selon la revendication 1, ledit copolymère contenant de 1 à 19% en poids de motifs diméthacrylate d'éthylène glycol et dans lequel les motifs polyvinylimidazolium présentent une valeur moyenne de n de 25 à 35 et dans lequel R est un radical alkyle ayant de 1 à 3 atomes de carbone.

5. Copolymère de vinylimidazole quarternisé-diméthacrylate d'éthylène glycol selon la revendication 1, ledit copolymère contenant de de 2 à 5% en poids de motifs diméthacrylate d'éthylène glycol.

6. Copolymère de vinylimidazole quarternisé-diméthacrylate d'éthylène glycol selon la revendication 5 dans lequel R est un radical méthyle.

7. Copolymère de vinylimidazole quarternisé-diméthacrylate d'éthylène glycol selon la revendication 6 dans lequel les motifs polyvinylimidazolium présentent une valeur moyenne de n de 28 à 32.

8. Copolymère de vinylimidazole quarternisé-diméthacrylate d'éthylène glycol selon la revendication 7 dans lequel $X^-$ est un ion halogénure.

9. Copolymère de vinylimidazole quarternisé-diméthacrylate d'éthylène glycol selon la revendication 8 dans lequel les motifs polyvinylimidazolium présentent une valeur moyenne de n de 30.

10. Copolymère de vinylimidazole quarternisé-diméthacrylate d'éthylène glycol selon la revendication 9 dans lequel $X^-$ est un ion chlorure.

11. Un procédé pour préparer le copolymère des revendications 1 à 10 caractérisé en ce que:

a) on fait réagir du monomère 1-vinylimidazole et du monomère diméthacrilate d'éthylène glycol en présence d'un catalyseur de polymérisation adéquat dans des conditions de polymérisation telles qu'on forme un produit de polymérisation initial.

b) on quaternise ledit produit de polymérisation initial pour former ledit copolymère.

12. Un procédé selon la revendication 11, dans lequel ledit catalyseur de polymérisation est le peroxyde de benzoyle.

13. Un composé selon l'une quelconque des revendications 1 à 10 pour utilisation comme médicament pour piéger les acides biliaires d'un mammifère ou pour abaisser le taux de cholestérol du sang d'un mammifère ou pour traiter la diarrhée d'un mammifère.

14. Un composé pharmaceutique selon la revendication 13, pour utilisation comme médicament pour piéger les acides biliaires ou pour abaisser le taux de cholestérol du sang, dans lequel ledit copolymère contient de 15 à 20% en poids de motifs diméthacylate d'éthylène glycol.

15. Un composé selon la revendication 14, dans lequel ledit copolymère contient 17% en poids de motifs diméthacrylate d'éthylène glycol.

16. Une composition pharmaceutique comprenant au moins l'un des composés des revendications 1 à 10 éventuellement avec des adjuvants et/ou supports pharmaceutiques.

17. La composition pharmaceutique selon la revendication 16, sous forme de dosage unitaire pour piéger les acides biliaires chez un mammifère, ladite composition comprenant un dosage de 2 à 5 grammes d'un copolymère selon la revendication 2 ou 3.

18. La composition pharmaceutique selon la revendication 16, sous forme de dosage unitaire pour abaisser le taux de cholestérol d'un mammifére, ladite composition comprenant un dosage de 2 à 5 grammes d'un copolymère selon la revendication 2 ou 3.

19. Une composition pharmaceutique selon la revendication 16, pour empêcher la diarrhée chez un mammifère ladite composition comprenant un copolymère selon la revendication 4 ou la revendication 5 ou la revendication 10.

**Revendications pour l'Etat contractant: AT**

1. Un procédé pour la préparation de coplymères de vinylimidazole quarternisé-diméthacrylate d'éthylène glycol comprenant de 1 pour cent à 25 pour cent en poids de motifs diméthacrylate d'éthylène glycol, le reste étant constitué par des motifs polyvinylimidazolium de formule structurelle.

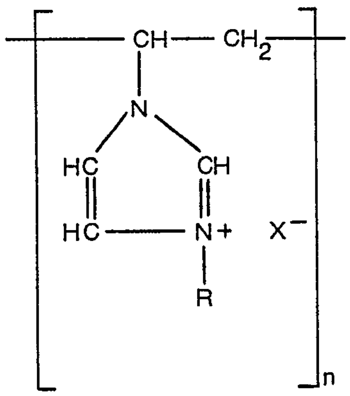

dans laquelle:

n, en moyenne, est de l'ordre de 3 à 40 et

R est choisi dans le groupe formé par les radicaux alkyle et alkényle possédant de 1 à 18 atomes de carbone et $—CH_2R^1$ dans laquelle $R^1$ est choisie dans le groupe formé par les radicaux phényle $—CH_2OH$, $—CH_2NHCOC_6H_5$, et $—CH_2NR^2R^3$ dans lesquels $R^2$ et $R^3$ sont chacun choisis dans le groupe formé par les radicaux méthyle et éthyle et dans lesquels $X^-$ est un anion, caractérisé en ce que

a) on fait réagir du monomère 1-vinylimidazole et du monomère diméthacrilate d'éthylène glycol en présence d'un catalyseur de polymérisation adéquat dans des conditions de polymérisation telles qu'on forme un produit de polymérisation initial.

b) on quaternise ledit produit de polymérisation initial pour former ledit copolymère.

2. Le procédé selon la revendication 1 pour préparer un copolymère de vinylimidazole quarternisé-diméthacrylate d'éthylène glycol caractérisé en ce que ledit copolymère contient de 15 à 20% en poids de motifs diméthacrylate d'éthylène glycol et dans lequel les motifs polyvinylimidazolium présentent une valeur moyenne de n de 3 à 10 et dans lequel R est un radical alkényle ayant de 2 à 4 atomes de carbone.

3. Le procédé selon la revendication 1 pour préparer un copolymère de vinylimidazole quarternisé-diméthacrylate d'éthylène glycol caractérisé en ce que ledit copolymère contient 17% en poids de motifs diméthacrylate d'éthylène glycol et dans lequel les motifs polyvinylimidazolium présentent une valeur moyenne de n de 5 et dans lequel R est un radical propyl-2-ène-1-yle, et $X^-$ est l'ion chlorure.

4. Le procédé selon la revendication 1 pour préparer un copolymère de vinylimidazole quarternisé-diméthacrylate d'éthylène glycol caractérisé en ce que ledit copolymère contient de 1 à 19% en poids de motifs diméthacrylate d'éthylène glycol et dans lequel les motifs polyvinylimidazolium présentent une valeur moyenne de n de 25 à 35 et dans lequel R est un radical alkyle ayant de 1 à 3 atomes de carbone.

5. Le procédé selon la revendication 1 pour préparer un copolymère de vinylimidazole quarternisé-diméthacrylate d'éthylène glycol caractérisé en ce que ledit copolymère contient de de 2 à 5% en poids de motifs diméthacrylate d'éthylène glycol.

6. Le procédé selon la revendication 5 pour préparer un copolymère de vinylimidazole quarternisé-diméthacrylate d'éthylène glycol caractérisé en ce que R est un radical méthyle.

7. Le procédé selon la revendication 6 pour préparer un copolymère de vinylimidazole quarternisé-diméthacrylate d'éthylène glycol caractérisé en ce que les motifs polyvinylimidazolium présentent une valeur moyenne de n de 28 à 32.

8. Le procédé selon la revendication 7 pour préparer un copolymère de vinylimidazole quarternisé-diméthacrylate d'éthylène glycol caractérisé en ce que $X^-$ est ion halogénure.

9. Le procédé selon la revendication 8 pour préparer un copolymère de vinylimidazole quarternisé-diméthacrylate d'éthylène glycol caractérisé en ce que les motifs polyvinylimidazolium présentent une valeur moyenne de n de 30.

10. Le procédé selon la revendication 9 pour préparer un copolymère de vinylimidazole quarternisé-diméthacrylate d'éthylène glycol caractérisé en ce que $X^-$ est ion chlorure.

11. Un procédé selon l'une quelconque d'un procédé de 1 à 10, caractérisé en ce que ledit catalyseur de polymérisation est le peroxyde de benzoyle.

12. Un procédé selon l'une quelconque des revendications 1 à 11 pour préparer un composé pharmaceutique pour utilisation comme médicament pour piéger les acides biliaires ou pour abaisser le taux de cholestérol du sang, caractérisé en ce que ledit copolymère contient de 15 à 20% en poids de motifs diméthacrylate d'éthylène glycol.

13. Le procédé selon la revendication 12 pour préparer une composition pharmaceutique caractérisé en ce que ledit copolymère contient 17% en poids de motifs diméthacrylate d'éthylène glycol.

14. Un procédé pour préparer une composition pharmaceutique caractérisé en ce qu'il comprend au moins l'un des composés des revendications 1 à 10 éventuellement avec des adjuvants et/ou supports pharmaceutiques.

15. Le procédé selon la revendication 14 pour préparer une composition pharmaceutique sous forme de dosage unitaire pour piéger les acides biliaires chez un mammifère caractérisé en ce que ladite composition comprend un dosage de 2 à 5 grammes d'un copolymère selon la revendication 2 ou 3.

16. Le procédé selon la revendication 14 pour préparer une composition pharmaceutique sous forme de dosage unitaire pour abaisser le taux de cholestérol du sang d'un mammifère, caractérisé en ce que ladite composition comprend un dosage de 2 à 5 grammes d'un copolymère selon la revendicaiton 2 ou 3.

17. Le procédé selon la revendication 14 pour préparer une composition pharmaceutique pour empêcher la diarrhée chez un mammifère caractérisé en ce que ce la composition comprend un copolymère préparé selon la revendication 4 ou la revendication 5 ou la revendication 10.